# DEMANDE DE BREVET EUROPEEN

(11) **EP 0 719 557 A1**
(43) Date de publication de la demande: **03.07.1996**
(21) Numéro de dépôt: 95402743.9
(22) Date de dépôt: 06.12.1995
(51) Int. Cl.: A61K 31/435

(54) **Utilisation de dérivés d'imidazopyridines pour la préparation de médicaments utiles dans le traitement des neuropathies périphériques**

(30) Priorité: 12.12.1994 FR 9414900
(71) Demandeur: SYNTHELABO, F-92350 Le Plessis Robinson (FR)
(72) Inventeur: Benavides, Jésus, F-92290 Chatenay Malabry (FR); George, Pascal, F-78730 St Arnoult en Yvelines (FR); Ferzaz, Badia, F-92160 Antony (FR); Scatton, Bernard, F-91120 Villebon sur Yvette (FR)
(74) Mandataire: Thouret-Lemaitre, Elisabeth

(57) **Abrégé**

Utilisation des composés répondant à la formule (I) pour la préparation de médicaments utiles dans la prévention et le traitement des neuropathies périphériques. 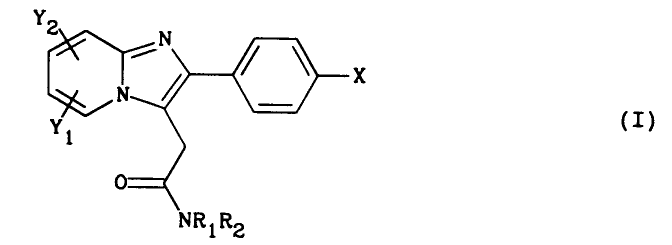
dans laquelle :
X représente un atome d'hydrogène ou d'halogène ou un groupe C₁₋₄ alkyle, C₁₋₄ alcoxy, trifluorométhyle, méthylthio, nitro ou amino,
Y₁ représente un atome d'hydrogène ou d'halogène ou un groupe C₁₋₄ alkyle,
Y₂ représente un groupe hydroxy ou C₁₋₄ alcoxy pouvant être substitué par un groupe C₃₋₆ cycloalkyle, ou un groupe SR dans lequel R est un atome d'hydrogène ou un groupe C₁₋₄ alkyle ou (4-alcoxyphényl)méthyle, et
R₁ et R₂ représentent, indépendamment l'un de l'autre, des atomes d'hydrogène ou des groupes C₁₋₄ alkyle linéaire ou ramifié.

## Description

La présente invention a pour objet l'utilisation de dérivés d'imidazopyridines pour la préparation de médicaments utiles dans la prévention et le traitement des neuropathies périphériques.

Les composés utiles selon la présente invention sont en partie décrits dans le brevet EP 0289371 et répondent à la formule suivante (I) 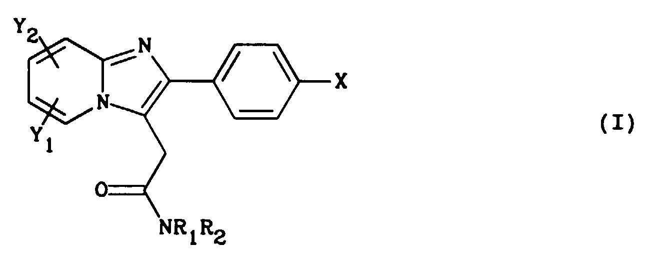 dans laquelle :
X représente un atome d'hydrogène ou d'halogène ou un groupe C₁₋₄ alkyle, C₁₋₄ alcoxy, trifluorométhyle, méthylthio, nitro ou amino,
Y₁ représente un atome d'hydrogène ou d'halogène ou un groupe C₁₋₄ alkyle,
Y₂ représente un groupe hydroxy ou C₁₋₄ alcoxy pouvant être substitué par un groupe C₃₋₆ cycloalkyle, ou un groupe SR dans lequel R est un atome d'hydrogène ou un groupe C₁₋₄ alkyle ou (4-alcoxyphényl)méthyle, et
R₁ et R₂ représentent, indépendamment l'un de l'autre, des atomes d'hydrogène ou des groupes C₁₋₄ alkyle linéaire ou ramifié.

Parmi ces composés les composés préférés sont ceux pour lesquels
Y₁ est en position 6 et représente un atome de chlore et Y₂ est un radical C₁₋₄ alcoxy pouvant être substitué par un radical C₃₋₆ cycloalkyle, et en particulier ceux pour lesquels Y₂ est en position 8.

Les composés de formule (I) ont été soumis à des essais pharmacologiques qui ont mis en évidence leurs propriétés neurotrophes dans un modèle de lésion locale du nerf sciatique chez le rat.
La lésion par congélation détruit les fibres du nerf sciatique qui subissent une dégénérescence wallérienne au site de la lésion et dans tout le tronçon distal ; elle conserve les gaines nerveuses et permet une régénération nerveuse dans des conditions reproductibles. Le processus de régénération commence à partir du côté proximal dans les heures qui suivent la lésion. On mesure la vitesse de régénération des fibres sensitives par un test de pincement ("pinch-test") réalisé 8 jours après la lésion.
On anesthésie au pentobarbital sodique à la dose de 60 mg/kg ip, des rats mâles de souche Sprague-Dawley (Iffa Credo) pesant environ 200 g. On incise la peau de la cuisse au niveau des biceps fémoraux puis on écarte les muscles lateralis et le biceps femoris afin de dégager le nerf sciatique. On repère le point de lésion par une micro suture sur le périnèvre au-dessus de la trifurcation du nerf sciatique. On réalise la lésion sur 1 mm par 6 cycles de congélation-décongélation à l'aide d'une cryode en cuivre pré-refroidie dans de l'azote liquide. On referme la plaie et on surveille les animaux quotidiennement. On répartit alors les animaux en trois lots de dix individus :
- un lot d'animaux traités par le véhicule (Tween 80 ™ à 0,1 %) administré 2 fois par jour par voie intrapéritonéale,
- un lot d'animaux traités par le composé à tester administré 2 fois par jour par voie intrapéritonéale à la dose de 1 mg/kg.

Huit jours après l'opération, on anesthésie légèrement les animaux et on remet le nerf sciatique à nu avant de le soumettre au "pinch-test". Dans ce test on pince légèrement le nerf sciatique à l'aide d'un forceps, en commençant par la région la plus distale et en remontant tous les 0,5 mm jusqu'au point où on obtient une réponse réflexe. La réponse se manifeste par une contraction des muscles de la région postérieure de l'animal, indiquant la zone d'arrivée du front des fibres sensitives régénérées. On marque ce point par une micro suture, on prélève le nerf et on mesure la distance entre le site de la lésion et la micro suture sous microscope à l'aide d'un papier millimétré.

Les résultats de ces essais montrent que les composés utilisés selon l'invention possèdent une activité neurotrophe (augmentation ≥ 10 % par rapport au groupe témoin) et peuvent être utilisés pour la préparation de médicaments utiles dans la prévention et le traitement des désordres neurologiques où les facteurs neurotrophes endogènes sont absents ou en quantité insuffisante pour induire la régénération axonale et où l'apport de substances capables de reproduire les effets des agents neurotrophes naturels en stimulant la régénération des neurones endommagés est nécessaire.

En particulier le 6-chloro-2-(4-chlorophényl)-8-méthoxy-*N,N*-dipropyl-imidazo[1,2-*a*]pyridine-3-acétamide peut être utilisé pour la préparation de médicaments utiles dans la prévention et le traitement des neuropathies périphériques.

Les composés peuvent donc être utilisés selon l'invention pour la préparation de médicaments utiles dans la prévention et le traitement des neuropathies périphériques telles que les neuropathies traumatiques (section ou écrasement d'un nerf) , ischémiques, métaboliques (diabète, urémie), infectieuses, alcooliques, médicamenteuses et les neuropathies génétiques etc. et pour celle de médicaments utiles dans le traitement des atteintes du motoneurone telles que l'amyotrophie spinale et la sclérose latérale amyotrophique.

Ils peuvent également être utilisés pour la préparation de médicaments utiles pour la prévention et le traitement des rétinopathies de diverses origines.

A cette fin ils peuvent être utilisés seuls ou en association avec d'autres substances thérapeutiques comme par exemple des agents diminuant la pression intraoculaire dans le cas du glaucome ou des agents anticancéreux en vue de réduire les effets secondaires de ces derniers.

A cet effet ils peuvent être présentés sous toutes formes pharmaceutiques adaptées à l'administration entérale ou parentérale, en association avec des excipients appropriés. Ils peuvent être présentés par exemple sous forme de comprimés, dragées, gélules, capsules, suppositoires, solutions ou suspensions buvables ou injectables ou patchs. Ces formes pharmaceutiques sont dosées pour permettre une administration journalière de 1 à 1000 mg de substance active.

## Revendications

1. Utilisation des composés répondant à la formule (I) pour la préparation de médicaments utiles dans la prévention et le traitement des neuropathies périphériques, 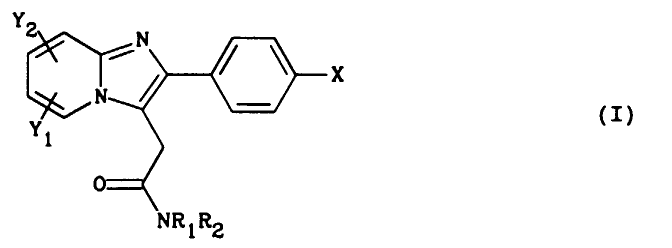 dans laquelle :
X représente un atome d'hydrogène ou d'halogène ou un groupe C₁₋₄ alkyle, C₁₋₄ alcoxy, trifluorométhyle, méthylthio, nitro ou amino,
Y₁ représente un atome d'hydrogène ou d'halogène ou un groupe C₁₋₄ alkyle,
Y₂ représente un groupe hydroxy ou C₁₋₄ alcoxy pouvant être substitué par un groupe C₃₋₆ cycloalkyle, ou un groupe SR dans lequel R est un atome d'hydrogène ou un groupe C₁₋₄ alkyle ou (4-alcoxyphényl)méthyle, et
R₁ et R₂ représentent, indépendamment l'un de l'autre, des atomes d'hydrogène ou des groupes C₁₋₄ alkyle linéaire ou ramifié.

2. Utilisation selon la revendication 1, caractérisée par le fait que les composés de formule (I) comportent comme radical Y₁ en position 6 un atome de chlore et comme radical Y₂ un groupe C₁₋₄ alcoxy pouvant être substitué par un groupe C₃₋₆ cycloalkyle.

3. Utilisation selon la revendication 1 ou la revendication 2, caractérisée par le fait que les composés de formule (I) comportent le radical Y₂ en position 8.

4. Utilisation du 6-chloro-2-(4-chlorophényl)-8-méthoxy-*N,N*-dipropyl-imidazo[1,2-*a*]pyridine-3-acétamide pour la préparation de médicaments utiles dans la prévention et le traitement des neuropathies périphériques.
